# EUROPÄISCHE PATENTSCHRIFT

(11) **EP 2 336 292 B1**
(45) Veröffentlichungstag und Bekanntmachung des Hinweises auf die Patenterteilung: **06.05.2015**
(21) Anmeldenummer: 09180007.8
(22) Anmeldetag: 18.12.2009
(51) Int. Cl.: C12M 1/12, C12M 1/34, C12M 1/02, C12M 1/00, C12P 5/02, C02F 3/28, C02F 11/04, C12M 1/107

(54) **Vorrichtung und Verfahren zur Vergärung von niederviskosen Materialien**
Method and device for fermenting low viscosity material
Dispositif et procédé de fermentation de matériaux peu visqueux

(43) Veröffentlichungstag der Anmeldung: 22.06.2011
(73) Patentinhaber: HF Biotec Berlin GmbH, 10117 Berlin (DE)
(72) Erfinder: Follmann, Heinrich, 10117 Berlin (DE); Hillbrecht, Berit, 15366 Hoppegarten (DE); Schubert, Christian, 10551 Berlin (DE); Martin, Torsten, 10247 Berlin (DE); Grahlmann, Rudolf, 10369 Berlin (DE)
(74) Vertreter: Gulde & Partner

(56) Entgegenhaltungen:
- WO-A1-81/02308
- WO-A1-2005/042416
- WO-A1-2008/025098
- DE-A1- 3 117 638
- DE-A1- 10 001 107
- FR-A1- 2 924 441

## Beschreibung

Die Erfindung betrifft eine Vorrichtung und ein Verfahren zur effizienten anaeroben Vergärung von flüssigen oder niederviskosen Materialien mit einem relativ geringen Anteil an Trockenmasse, wie z.B. Faserstoffen, wobei die aktive Biomasse vollständig im Reaktor zurückgehalten oder in diesen zurückgeführt wird. Dabei wird durch die Einstellung unterschiedlicher Temperaturen eine Kompartimentierung eines Gärbehälters in zwei unterschiedliche Reaktionsräume erreicht.

Mit der staatlichen Förderung erneuerbarer Energien gelangen Biogasanlagen immer weiter in das öffentliche Interesse. Neben der Fermentation nachwachsender Rohstoffe, widmet man sich heute vermehrt auch der Fermentation Kohlenstoffreicher "Abfallprodukte", wie z.B. Gülle, Melasse, Vinasse o. a. dünnflüssigen Medien.

Im Stand der Technik sind verschiedene Vorrichtungen, d.h. Biogasfermenter beschrieben worden, die im Wesentlichen das Ziel verfolgen, organische Masse durch anaerobe mikrobiologische Prozesse mit hohen Stoffumsatzraten zu Biogas umzusetzen. Je nach Bauart des Fermenters und in Abhängigkeit des umzusetzenden Substrats sind dabei unterschiedlich lange Verweilzeiten erforderlich.

Man unterscheidet zwischen kontinuierlichen und diskontinuierlich arbeitenden Fermentertypen, wobei sich in Europa im Wesentlichen die kontinuierlich arbeitenden Fermenter wegen der höheren Effizienz durchgesetzt haben.

Beispielsweise sind Rührkesselfermenter wegen des einfachen Behälterbaus preiswert zu erstellen. In den meist zylindrisch geformten Behältern wird der Inhalt mit Hilfe eines hydraulischen Rührwerks durchmischt. Dabei kann allerdings nicht ausgeschlossen werden, dass auch Frischsubstrat mit in den Auslauf gelangt. Zudem ist der Energieaufwand der zum Durchmischen des Substrats eingesetzt wird, relativ hoch. Derartige Fermenter werden zumeist für Substrate aus nachwachsenden Rohstoffen eingesetzt.

Für schwierigere Substrate, insbesondere für Substrate mit hohem Feststoffanteil, ist beispielsweise der liegend ausgeführte Pfropfenstromreaktor besser geeignet. Dieser wird nur vertikal durchmischt und in der Regel ist kein Kurzschluss zwischen Ein- und Auslauf möglich. Des Weiteren sind noch so genannte Garagenfermenter sowie die Lagunentechnologie zu erwähnen.

Bei all diesen Technologien sind lange Aufenthaltszeiten, d.h. länger als 30 Tage, erforderlich, da ein Teil der aktiven Biomasse mit dem Substrat ausgetragen wird. Insbesondere die methanogenen Bakterien verfügen jedoch über recht geringe Wachstumsraten, so dass die Bakterienkonzentration im Reaktor insgesamt relativ niedrig ist, wodurch die für den Stoffumsatz erforderlichen hohen Verweilzeiten begründet sind.

Mit wesentlich kürzeren Verweilzeiten arbeiten die UASB-Reaktoren (Upflow Anaerobic Sludge Blanket-Reaktoren), welche zumeist für die anaerobe Behandlung von kommunalen Abwässern eingesetzt werden. In diesen Reaktoren findet eine Biomasseanreicherung in Form von Belebtschlamm statt, welcher pelletartig aggregiert und durch eine im oberen Teil angebrachte Separationseinrichtung zurückgehalten wird. Alternativ kann der Ablauf des Reaktors mit Druck beaufschlagt werden, was zur Folge hat, dass eine Sedimentation der Biomasse erfolgen kann, wobei das Sediment in den Reaktor zurückgeführt wird. Die Durchmischung erfolgt durch die bei der Gärung entstehenden Gase, die in Bläschenform an den Schlammpartikeln haften oder aufsteigen.

Alternativ wurde in der DE 10 2005 024 886 B3 versucht, die mikrobielle Biomasse mit magnetisch wirkenden Partikeln zu versetzen und diese aus dem Ablauf mit Hilfe einer Magnetkraft-Rückhalteeinrichtung wieder abzuscheiden. Dabei wird auch ein geringer Teil der Biomasse mit abgeschieden und kann gemeinsam mit den Magnetpartikeln in den Biogasfermenter zurückgeführt oder in einen weiteren Fermenter eingebracht werden.

Derartige Reaktoren sind allerdings nur für Substrate mit hohem Trockenmassen (TS)-gehalt geeignet, da ansonsten die für eine vollständige Vergärung benötigten Standzeiten zu lang werden. Auf Grund der geringen aktiven Biomasse werden zudem extrem große Reaktorvolumina benötigt, was häufig zu einer negativen Energiebilanz führt.

Zur Steigerung der aktiven Biomasse wird die aus der Abwassertechnik bekannte Festbett-Technologie auch auf Anaerobreaktoren übertragen. Hierbei werden Besiedlungssubstrate in den Reaktor eingebracht, auf denen die Bakterien aufwachsen.

Beispielsweise beschreibt die DE 44 15 017 C2 einen zweistufigen Kombi-Biogasreaktor zur Aufarbeitung von Gülle. In der ersten Stufe, der Hydrolyse des Substrats, wird die Gülle einer trichterförmigen Sammelstufe zugeführt. Hier wird die Gülle aufgeheizt, dadurch fließt die Gülle von unten nach oben. Zur Verhinderung von Schwimmdecken, die im Extremfall zu Verstopfungen des Systems führen, sind im Reaktor Rührvorrichtungen angebracht.

Anschließend strömt das Substrat von oben nach unten durch einen Glasfaserfilter. Grobteile sinken schneller ab und führen am Boden des Reaktors zu einer Schlammablagerung. Etwa 15 % dieses Schlammes werden zum Animpfen des Frischsubstrats in den Kreislauf zurückgeführt.

Allerdings weist dieses System einige Nachteile auf. Beispielsweise bestehen die gröberen Partikel in der Gülle, die zu Boden sinken und das Schlammbett bilden, aus Stroh- bzw. Futterresten. Diese Anteile verfügen über einen hohen Anteil an Lignin/ Lignocellulose, welches kaum bzw. nur sehr schwer im Biogasprozess abgebaut werden kann. Es wird also überwiegend Inertmaterial zum Animpfen dem Frischsubstrat zugeführt, so dass auf diesem nicht aufgeschlossenen Material auch vergleichsweise wenig aktive Biomasse siedelt, d.h. dass die Bakterienrückführung sehr uneffizient ist. Zudem verbrauchen die eingesetzten Rührer und Heizungen Energie, so dass die Nettoenergieausbeute des Systems deutlich herabgesetzt wird. Nicht zuletzt ist die Bauart des Biogasreaktors sehr aufwendig, was zu hohen Erstinvestitionen und Unterhaltungskosten sowie einem hohen Wartungsaufwand führt.

WO 2008/025098 A1 beschreibt einen zylindrischen Bioreaktor und ein Verfahren zum Vergären niederviskoser Stoffe. Der Bioreaktor ist ein Festbettreaktor mit Aufwüchsträger und Rezirkulationkreis.

WO2005/042416 A1 beschreibt einen Festbettreaktor zur anaeroben Abwasserbehandlung mit Rezirkulationskreis und Wärmeaustaucher außerhalb des Reaktors.

WO81/02308 A1 beschreibt einen Reaktor zur Vergärung von flüssigen organischen Materialien, der durch einen Rezirkulationskreis mit einem Feststoffabschneider verbunden ist.

Es ist somit die Aufgabe der vorliegenden Erfindung einen Bioreaktor zur Verfügung zu stellen, der für Substrate mit niedrigem TS-Gehalt geeignet ist, und die Nachteile im Stand der Technik überwindet. Insbesondere soll ein Reaktor in einfacher Bauart zur Verfügung gestellt werden, der mit möglichst wenig extern zugeführter Energie betrieben werden kann. Darüber hinaus soll das System eine hohe Effektivität aufweisen, so dass die Verweildauer im Reaktor auf wenige Tage begrenzt werden kann und der Betreuungsaufwand während der Standzeit minimal wird.

Die erfindungsgemäße Aufgabe wird mit einer Vorrichtung und einem Verfahren gemäß den unabhängigen Ansprüchen gelöst. Bevorzugte Ausgestaltungen sind in den abhängigen Ansprüchen beschrieben.

Die Erfindung betrifft also eine Vorrichtung zur Vergärung von niederviskosen organischen Materialien aufweisend einen Bioreaktor und mindestens einen überstauten Feststoffabscheider. Dabei weist der Bioreaktor mindestens die folgenden Bauteile auf, und zwar einen Reaktorbehälter mit einem konusförmigen Reaktorboden, Seitenwänden und einer Reaktordecke, wobei im Inneren des Reaktorbehälters mindestens eine Aufnahme zur Befestigung einer Vielzahl von Aufwuchsträgern im Wesentlichen parallel zur Reaktordecke und im mittleren Drittel des Reaktorbehälters angeordnet ist, und wobei der Bereich vom konusförmigen Boden bis zur mindestens einen Aufnahme einen ersten Reaktionsraum und der Bereich von der mindestens einen Aufnahme bis zur Reaktordecke einen zweiten Reaktionsraum bildet; eine Vielzahl von Aufwuchsträgern, die von der Reaktordecke bis zur mindestens einen Aufnahme gespannt sind; mindestens eine Substratzufuhr im Bereich des ersten Reaktionsraumes, wobei die Substratzufuhr einen ersten Wärmetauscher zur Temperierung des sich in der Substratzufuhr befindenden Substrats aufweist; mindestens ein Rezirkulationsmittel, das geeignet ist, Rezirkulat aus dem zweiten Reaktionsraum in den erste Reaktionsraum zu überführen, wobei das Rezirkulationsmittel einen zweiten Wärmetauscher zur Temperierung des sich in dem Rezirkulationsmittel befindenden Rezirkulats aufweist; und mindestens einen Überlauf, der im Bereich des zweiten Reaktionsraums zwischen der Reaktordecke und dem Rezirkulationsmittel angeordnet ist. Der mindestens eine überstaute Feststoffabscheider weist mindestens einen konusförmigen Boden auf, wobei an der Spitze des Konus ein Feststoffentnahmemittel angeordnet ist, das mit der Substratzufuhr des Bioreaktors verbunden ist; und mindestens einen Zulauf, der mit dem Überlauf des Bioreaktors verbunden ist, wobei der überstaute Feststoffabscheider auf Höhe oder unterhalb des Reaktorbodens neben dem Bioreaktor angeordnet ist.

Die Erfindung betrifft also eine Vorrichtung zur effizienten anaeroben Vergärung von flüssigen oder niederviskosen organischen Materialien mit einem relativ geringen Anteil an Trockenmasse, wie z.B. Faserstoffen, wobei die aktive Biomasse effizient im Reaktor zurückgehalten wird und wenig externe Energie zugeführt werden muss. Bei dem erfindungsgemäßen Reaktor handelt es sich um einen kompartimentierten Reaktor mit Festbetttechnologie, welcher zielgerichtet aufgeteilt ist in einen unteren Bereich, d.h. einen ersten Reaktionsraum, mit Bevorzugung der rasch ablaufenden hydrolytischen Vergärung mit sich unmittelbar anschließender Acidogenese und einen oberen Bereich, d.h. einen zweiten Reaktionsraum, mit bevorzugter Acetogenese und Methanogenese, wobei im zweiten Reaktionsraum zur Erhöhung der aktiven Biomasse Aufwuchsträger angebracht sind.

Der Reaktor eignet sich besonders für Substrate mit geringen Trockenmasse (TS)-Gehalten, wie z.B. Gülle, Schlempe, Vinasse, Glycerin, Stärke oder Maisquellwasser und einem dabei nicht zu hohen Anteil an Inertmaterial, dem so genannten persistierenden CSB (chemischen Sauerstoffbedarf). Mit der Festbetttechnologie kann vorteilhafterweise ein Aufkonzentrieren der langsam wachsenden Methanbakterien im oberen Teil des Reaktors erreicht werden. Infolge der hohen Biomassedichte erhöhen sich dadurch die Abbauraten und damit wird die erforderliche Verweilzeit des Substrates im Reaktor vermindert. Gleichzeitig werden durch die Kompartimentierung zwei verschiedene Reaktionsräume geschaffen, die auf die Bedingungen der zwei verschiedenen Bakterienpopulationen angepasst werden können. Dabei wird im unteren Reaktionsraum eine geringere Temperatur und ein geringerer pH-Wert als im oberen Reaktionsraum eingestellt. Auf Grund des Temperaturunterschieds und der Befüllung von unten wird das zu vergärende Substrat von unten nach oben durch den Bioreaktor transportiert. Es ergibt sich dadurch ein primärer aufwärtsgerichteter Stoffstrom.

Mit der erfindungsgemäßen Vorrichtung können Verweilzeiten von 2 - 4 Tagen und Abbauraten von etwa 80 - 85 % bezogen auf die Trockenmasse erreicht werden, selbst wenn der Bioreaktor mit Schweinegülle betriebenen wird. Das bedeutet, die erfindungsgemäße Vorrichtung ermöglicht extrem kurze Verweilzeiten bei gleichzeitig hohem Stoffumsatz. Daher kommt die erfindungsgemäße Vorrichtung trotz extrem einfacher Bauweise auch mit kleinen Volumina aus.

Die erfindungsgemäße Vorrichtung besteht aus mindestens einem Bioreaktor und einem überstauten Feststoffabscheider. Dabei wird mittels des überstauten Feststoffabscheiders eine effektive Möglichkeit geschaffen, aktive Biomasse von dem Gärrest abzutrennen und dem Bioreaktor wieder zuzuführen. Je nach Volumen des überstauten Feststoffabscheiders und der Menge des anfallenden vergärten Überlaufs werden ein oder mehrere Feststoffabscheider mit dem Bioreaktor kombiniert. Bevorzugte Volumina des Feststoffabscheiders liegen im Bereich von 1 bis 10 m³, besonders bevorzugt im Bereich von 1 bis 5 m³.

Bei dem erfindungsgemäßen Bioreaktor handelt es sich um einen geschlossenen Behälter, mit einem konusförmigen Reaktorboden, Seitenwänden und einer Reaktordecke. Der Reaktorbehälter kann eine runde, ellipsoide oder vieleckige Grundfläche aufweisen. Vorzugsweise handelt es sich bei dem Behälter um einen Zylinder mit runder Grundfläche. Die Seitenwände sind im Wesentlichen vertikal, d.h. mit einem Winkel im Bereich von 80 bis 100°, vorzugsweise im Bereich von 85 bis 95° und am meisten bevorzugt im Bereich von 88 bis 92° zum Boden ausbildet, auf dem der Bioreaktor steht. Auf Grund des konusförmigen Reaktorbodens, steht der Bioreaktor nicht direkt auf dem Erdboden auf, sondern ist in einem Fußgestell gehalten. Das Fassungsvolumen des Reaktorbehälters liegt im Bereich von 1 m³ bis 4000 m³, vorzugsweise im Bereich von 100 m³ bis 1000 m³. Die Höhe des erfindungsgemäßen Reaktorbehälters wird derart gewählt, dass eine deutliche Kompartimentierung des Behälters in zwei Bereiche möglich ist und gleichzeitig, der Temperaturgradient ausreicht, um den Stofftransport zu ermöglichen. Bevorzugte Höhen liegen im Bereich von 1 m bis 30 m, vorzugsweise im Bereich von 3 m bis 20 m. Der erfindungsgemäße Reaktorboden ist konisch ausgestaltet, wobei sich der Konus über 5 bis 20 %, vorzugsweise 5 bis 15 % der Gesamthöhe des Reaktorbehälters erstreckt. Die Verjüngung des Konus erfolgt mit einer Steilheit, dass feste Teilchen des Substrats, insbesondere Inertmasse, wie Stroh und Futterreste in die Spitze des Konus sinken. In einer bevorzugten Ausgestaltung der Erfindung weist der Reaktorboden an der Spitze des Konus ein Feststoffentnahmemittel zur Entnahme des Gärrestes auf. Damit wird vorteilhafterweise nur das Inertmaterial entnommen, wobei die Biomasse dem Reaktor erhalten bleibt.

Erfindungsgemäß ist im Inneren des Reaktorbehälters mindestens eine Aufnahme zur Befestigung einer Vielzahl von Aufwuchsträgern angeordnet. Die Aufnahme ist dabei im Wesentlichen parallel zur Reaktordecke und im mittleren Drittel des Reaktorbehälters angeordnet, vorzugsweise im Bereich von 30 bis 60% von der Reaktordecke aus gesehen, weiter bevorzugt im Bereich von 30 bis 40% von der Reaktordecke aus gesehen, so dass sie eine Art Zwischenboden darstellt. Der Bereich vom konusförmigen Boden bis zur mindestens einen Aufnahme wird erfindungsgemäß als erster Reaktionsraum und der Bereich von der mindestens einen Aufnahme bis zur Reaktordecke als zweiter Reaktionsraum bezeichnet. Die erfindungsgemäße Aufnahme ist vorzugsweise derart ausgestaltet, dass der Substratfluss aus dem ersten Reaktionsraum in den zweiten Reaktionsraum nicht behindert wird. Vorzugsweise handelt es sich bei der Aufnahme um in einer Ebene angeordnete Träger, die Mittel zur Befestigung der Aufwuchsträger aufweisen. Geeignete Mittel zur Befestigung richten sich nach der Art der verwendeten Aufwuchsträger und sind dem Fachmann bekannt. Beispielweise können Haken, Löcher, Ösen, Klemm- oder Einspanvorrichtungen verwendet werden. In der Ausgestaltung mit einer Aufnahme ist die Vielzahl von Aufwuchsträgern an der Aufnahme und an der Reaktordecke befestigt.

In einer besonders bevorzugten Ausgestaltung der erfindungsgemäßen Vorrichtung weist der Bioreaktor eine zweite Aufnahme auf. Die zweite Aufnahme ist vorzugsweise unterhalb der Reaktordecke, d.h. im Bereich von 0 bis 20 % der Höhe des Reaktors von der Reaktordecke aus, vorzugsweise im Bereich von 5 bis 20 %, weiter bevorzugt im Bereich von 10 bis 20 % des Reaktors von der Reaktordecke aus angeordnet. In dieser Ausgestaltung der Erfindung ist die Vielzahl an Aufwuchsträgern zwischen den beiden Aufnahmen gespannt.

Vorzugsweise handelt es sich bei den Aufwuchsträgern um Folien, besonders bevorzugt um Kunststofffolien. Geeignete Kunststoffe aus denen die Aufwuchsträger hergestellt werden, sind Polyethylen, Polypropylen, Polystyrol, Polyvinylchlorid, Polyamid oder Polyimid, besonders bevorzugt bestehen die Kunststofffolien aus Polyethylen oder Polypropylen. Vorteilhafterweise werden die Folien zwischen den Aufnahmen oder der einen Aufnahme und der Reaktordecke segeltuchförmig in den zweiten Reaktionsraum des Reaktors aufgehängt. Dabei werden die Folien nur so straff gespannt, dass noch ein Schwingen und Bewegen der Folien gegeneinander möglich ist. Der Abstand der Aufwuchsträger zueinander liegt im Bereich von 3 bis 20 cm, vorzugsweise im Bereich von 5 bis 15 cm.

Die Aufwuchsträger dienen zur Ansiedelung und räumlichen Lokalisierung der langsam wachsenden methanogenen Bakterien. Allerdings wird vorteilhafterweise mit den erfindungsgemäßen Aufwuchsträgern, der sonst übliche dicke Bewuchs mit Bakterien am Aufwuchsträger verhindert. Durch die flexible Ausführung des Trägermaterials, das selbst flexibel ist und biegsame Strukturen enthält wird im Falle eines etwaigen Zuwachsens zwischen zwei benachbarten Aufwuchsträgern die überschüssige und meist inaktive Biomasse durch Verbiegung und/oder Pendelbewegung des Trägermaterials und die damit verbundene Reibungsenergie regelmäßig und zuverlässig in den unteren konischen Teil des Reaktors abgeworfen. Dadurch wird das Verhältnis von aktiver zu passiver Biomasse deutlich erhöht, da nur die Bakterien in der obersten Schicht des Aufwuchses ausreichend mit Nährstoffen versorgt werden und daher am aktiven Stoffumsatz teilnehmen können. Ferner werden die sonst bei Festbettreaktoren auftretenden Verstopfungen zuverlässig verhindert.

Dadurch, dass die Biomasse in regelmäßigen Abständen von einzelnen Aufwuchsträgern abgeworfen wird und sich neu ansiedelt, ist auch bei relativ hohem Inertanteil der dem Reaktor zugeführten organischen Fracht stets sichergestellt, dass genügend aktive Biomasse vorhanden ist und sich in erheblichem Maße im oberen Teil des Reaktors anreichern kann. Dennoch wird vorteilhafterweise eine gewisse kritische Menge an aktiver Biomasse nie überschritten.

In einer bevorzugten Ausgestaltung der Erfindung werden die Oberflächen der Aufwuchsträger weiter veredelt. Beispielsweise können die Aufwuchsträger mikrostrukturiert und/oder mit organischen Substraten beschichtet werden. Durch die Behandlung der Oberflächen kann ein außergewöhnlich schneller Bewuchs zu Beginn der Beimpfungsphase mit den wichtigen Methanbildnern und anderen Mikroorganismen, d.h. während des Anfahrens des Reaktors, erreicht werden. Eine bevorzugte Mikrostrukturierung wird mittels einer Bestrahlung der Folien mit Kathodenstrahlung erreicht. Dabei wird vorzugsweise ein gepulster Strahl verwendet, um die Temperatur der Folien unterhalb der Schmelztemperatur zu halten. Vorzugsweise weisen die bestrahlten Folien konkave und konvexe Mikrostrukturen, d.h. Erhöhungen und Vertiefungen, auf. Eine geeignete Porengröße nach der Bestrahlung liegt vorzugsweise im Bereich von 50 bis 200 µm, besonders bevorzugt im Bereich von 100 bis 200 µm.

Die Beschichtung erfolgt mit organischen Substraten, vorzugsweise mit Substraten, die als Kohlenstoffquelle für die methanogenen Bakterien dienen. Vorzugsweise werden Zuckermonomere, -dimere und -oligomere verwendet. Besonders bevorzugt besteht die Beschichtung aus Stärke und/oder Stärkederivaten. Durch die Beschichtung der Oberflächen mit einem Anzuchtsubstrat kann die Besiedelung der Aufwuchsträger weiter beschleunigt werden.

Vorteilhafterweise sind die erfindungsgemäß veredelten, d.h. mikrostrukturierten und/oder beschichteten Kunststofffolien stabiler als herkömmliche Folien oder Textilien, d.h. sie reißen nicht ab, und werden von den im Reaktor ablaufenden Prozessen nicht zersetzt.

Der erfindungsgemäße Bioreaktor weist des Weiteren mindestens eine Substratzufuhr im Bereich des ersten Reaktionsraumes auf, wobei die Substratzufuhr einen ersten Wärmetauscher zur Temperierung des sich in der Substratzufuhr befindenden Substrats aufweist. Vorzugsweise ist die Substratzufuhr im Bereich von 10 bis 40%, vorzugsweise im Bereich von 10 bis 30% der Höhe des Reaktorbehälters bezogen auf den Reaktorboden angeordnet. Dies bedeutet, dass die Substratzufuhr in den ersten Reaktionsraum erfolgt, in dem Hydrolyse und Versäuerung stattfinden. Dabei sollte das Substrat zwar in Bodennähe, aber nicht vollständig am Boden zugeführt werden, um ein Absinken der schwereren Partikel, vorzugsweise der Inertmasse, noch zu gewährleisten und dadurch eine Vortrennung herbeizuführen. Besonders bevorzugt erfolgt die Substratzuführung im konischen Teil des Reaktorbehälters. In einer weiteren Ausgestaltung sind mehrere Substratzufuhren vorgesehen, die auf gleicher oder in unterschiedlichen Höhen in den Reaktorbehälter führen.

Die Erwärmung des kalten Substrates erfolgt durch einen speziellen Wärmetauscher, wobei das Substrat nur unvollständig, d.h. bewusst nicht auf das Niveau der eigentlichen Fermentationstemperatur im ersten Reaktionsraum erwärmt wird. Der Wärmetauscher erwärmt das kalte zugeführte Substrat vorzugsweise durch Kreuzen dieses Stromes mit dem warmen Fermentationsüberlauf. Der Fermentationsüberlauf kühlt sich dabei stark ab. Vorzugsweise wird die Abwärme des im Überlauf ablaufenden Gärrestes somit nahezu vollständig genutzt. Besonders bevorzugt kann die Abwärme des Rezirkulats mitgenutzt werden, wenn die Abwärme des Gärrestes im Überlauf nicht ausreicht. Dabei sollte allerdings die Temperatur des Rezirkulats nicht unter die Temperatur des eingehenden Substrats fallen und die Prozessführung und Bauart der Vorrichtung muss derart erfolgen, dass eine selbstständige Temperaturregelung des Rezirkulats möglich bleibt. Sollte die Abwärme aus Überlauf und Rezirkulat nicht ausreichen, wie beispielsweise, wenn das zugeführte Substrat, beispielsweise im Winter, im Substratlager kälter als üblich wird oder in der Anfahrphase des Reaktors, so kann extern zugeheizt werden. Dazu weist der Wärmetauscher optional noch ein Heizelement auf. Üblicherweise kann der erfindungsgemäße Bioreaktor allerdings ohne zusätzliche oder nur mit minimaler externer Energiezufuhr betrieben werden.

Das unterhalb des Überlaufs, d.h. in Richtung auf den Reaktorboden, angeordnete Rezirkulationsmittel ist geeignet, Rezirkulat, d.h. einen Teil des schon bereits vergorenen Substrats, aus dem zweiten Reaktionsraum in den ersten Reaktionsraum zurückzuführen. Dabei werden zum einen noch nicht vollständig abgebaute organische Verbindungen und aktive Biomasse in den Gärprozess zurückgeführt. Zudem können durch die Rezirkulation Schwimmschichten vermieden werden, wobei bei Gefahr der Bildung von Schwimmschichten die Umlaufzahl der Rezirkulation erhöht wird und umgekehrt. Der erfindungsgemäße Bioreaktor kann ein oder mehrere Rezirkulationsmittel aufweisen.

Das Rezirkulationsmittel weist ebenfalls einen Wärmetauscher zur Temperierung des sich in dem Rezirkulationsmittel befindenden Rezirkulats auf. Auch der Wärmetauscher des Rezirkulats kann noch ein zusätzliches Heizelement aufweisen. Durch den Einbau von zwei separat arbeitenden Wärmetauschern wird somit die Ausgangstemperatur des zugeführten Substrats unabhängig von der des Rezirkulats eingestellt. Dadurch kann sichergestellt werden, dass die Kompartimentierung des Reaktors in einen unteren Versäuerungsbereich (erster Reaktionsraum) und einen oberen Methanisierungsbereich (zweiter Reaktionsraum) erhalten bleibt. Beispielsweise kann bei Absinken der Temperatur in dem Versäuerungsbereich die Temperatur des Substratzulaufs erhöht oder bei Absinken der Temperatur in dem Methanisierungsbereich die Temperatur des Rezirkulats erhöht werden. Die Temperaturregelung d.h. insbesondere die Erwärmung des Fermenterinhalts, wird somit über die Erwärmung des zugeführten Substrates sowie des Rezirkulats gesteuert.

In einer bevorzugten Ausgestaltung weist das Rezirkulationsmittel ein Mittel zur Aufnahme des Rezirkulats und ein Mittel zur Abgabe des Rezirkulats auf. Das bedeutet, dass das Rezirkulat aus dem Reaktorbehälter abgeführt, im Rezirkulationsmittel transportiert und an anderer Stelle dem Reaktorbehälter wieder zugeführt wird. Dabei ist das Mittel zur Aufnahme im Bereich von 0 bis 20% der Höhe des Reaktorbehälters bezogen auf die Reaktordecke angeordnet, wobei das Mittel zur Aufnahme des Rezirkulats immer unterhalb des Überlaufs, d.h. in Richtung auf den Reaktorboden angeordnet. Das Mittel zur Abgabe des Rezirkulats ist im Bereich von 60 bis 80%, vorzugsweise im Bereich von 70 bis 80% der Höhe des Reaktorbehälters bezogen auf die Reaktordecke angeordnet. In einer bevorzugten Ausgestaltung der Erfindung weisen Substratzufuhr und das Mittel zur Abgabe des Rezirkulats eine gemeinsame Zuführung in den Reaktorbehälter auf. Alternativ werden Rezirkulat und Substrat parallel zugeführt.

Der erfindungsgemäße Überlauf ist im Bereich des zweiten Reaktionsraums zwischen der Reaktordecke und dem Rezirkulationsmittel angeordnet. Der Überlauf dient der Abfuhr von vergorenem Material, d.h. der Abfuhr des Gärgutes. Im einfachsten Fall handelt es sich bei dem Überlauf nur um eine Öffnung in der Seitenwand des Reaktorbehälters und ein sich daran anschließendes Fallrohr. Durchmesser und Länge des Fallrohrs bestimmen die Menge des abzuführenden Gärrestes. Vorzugsweise können in den Überlauf Mittel zur aktiven Abfuhr des Gärrestes sowie Mittel zum Unterbrechen der Abfuhr des Gärgutes integriert sein. Der Ausgang des Überlaufs führt direkt in den Zulauf des überstauten Feststoffabscheiders. Bei dem überstauten Feststoffabscheider handelt es sich um einen kleinen, ebenfalls konisch ausgeführten Behälter, der auf Höhe oder unterhalb des Reaktorbodens neben dem Bioreaktor, d.h. außerhalb des Bioreaktors, angeordnet ist. Vorzugsweise ist der überstaute Abscheider somit am tiefsten Punkt des Bioreaktors angeordnet. Auf Grund des geodätischen Höhenunterschiedes der vom oberen Teil des Fermenters in den tiefer gelegenen Abscheider laufenden Flüssigkeit entgasen die Partikel teilweise und konzentrieren sich im unteren Teil des Abscheiders auf.

Der überstaute Abscheider selbst ist im Wesentlichen ein konischer Behälter, der mit einem Zulauf und einem Ablauf ausgestattet ist. Zwischen Zulauf und Ablauf ist vorzugsweise eine Trennwand angeordnet, die vorzugsweise vom Deckel des Behälters bis maximal ²/₃ in den Behälter hineinreicht. Durch die Trennwand können vorteilhafterweise die Stoffströme von Zu- und Ablauf sauber getrennt werden. Überraschenderweise sinken die aktiven lebenden Zellen in dem überstauten Abscheider nach unten in den Konus ab, während die abgestorbene Biomasse oben schwimmt und wieder in den Ablaufstrom und damit in den Gärrest ausgetragen wird. An der Spitze des Konus des Abscheiders ist somit ein Feststoffentnahmemittel angeordnet, das mit der Substratzufuhr des Bioreaktors verbunden ist, und somit die noch lebende aktive Biomasse in den Bioreaktor zurückführt. Die im unteren Teil des überstauten Abscheiders aufkonzentrierten Mikroorganismen, die aus dem Überlauf des Reaktors stammen, werden somit in den Konus des Reaktors eingespeist und mit den dort bereits aufkonzentrierten Mikroorganismen vermischt. Auf diese Weise kann sichergestellt werden, dass überwiegend lebende aktive Biomasse in den Fermenter zurückgeführt wird. Dies führt zu einem wesentlich intensiveren Aufschluss der organischen Fracht, die somit in kürzerer Zeit und mit deutlich höheren Umsatzraten umgesetzt werden kann. Der hier beschriebene Effekt wird überlagert durch den ebenfalls vorteilhaften Effekt der Entgasung von kleinen Agglomeraten mit lebender Biomasse.

Durch die Kombination aus dem überstauten Abscheider, dem Abfall der Biomasse von den beweglichen Trägern und der damit verbundenen reaktorinternen Durchmischung der Bakterien, dem ebenfalls konisch ausgeführten unteren Teil des Reaktors, in dem sich aufgrund des Trichtereffektes ebenfalls Biomasse ansammelt und der Rezirkulation des Substrats, wodurch leichte organische Verbindungen und Biomasse in den Gärprozess zurückgeführt werden, wird die unerwartet gute Effektivität der erfindungsgemäßen Vorrichtung erreicht.

Im oberen, d.h. zweiten Reaktionsraum, bildet sich zwischen Reaktordecke und Überlauf vorzugsweise ein Gassammelraum aus. Dieser Gassammelraum ist frei von Gärgut und wird zum Auffangen und Sammeln des während der Gärung entstehenden Gases genutzt. In einer Ausgestaltung der Erfindung kann der Gassammelraum vergrößert werden, indem die Reaktordecke als Gashaube ausgestaltet ist. Dabei handelt es sich um ein dehnbares Material, so dass sich das Volumen der Menge des entstandenen Gases anpassen kann. Im Bereich des Gassammelraums ist vorzugsweise ein Gasentnahmemittel angeordnet. Geeignete Gasentnahmemittel sind dem Fachmann bekannt. Alternativ oder in Ergänzung zur integrierten Gashaube kann auch ein externer Gasspeicher mit der Vorrichtung, vorzugsweise über das Gasentnahmemittel, verbunden sein.

Die Erfindung betrifft weiterhin ein Verfahren zur Vergärung von niederviskosen organischen Materialien, wobei eine erfindungsgemäße Vorrichtung verwendet wird. Insbesondere handelt es sich bei dem erfindungsgemäßen Verfahren um eine anaerobe Vergärung.

Die Erfindung betrifft weiterhin ein Verfahren zur Vergärung von niederviskosen organischen Materialien, wobei das Verfahren die folgenden Schritte umfasst: Vorwärmen der niederviskosen organischen Materialien auf eine Temperatur unterhalb einer Temperatur im Bereich von 28 bis 33 °C, Einbringen der vorgewärmten niederviskosen organischen Materialien als Substrat in einen ersten Reaktionsraum eines Bioreaktors, wobei sich durch die von den im Substrat enthaltenen acetogenen Bakterien produzierte Gärungswärme im ersten Reaktionsraum die Temperatur auf die Temperatur im Bereich von 28 bis 33 °C erhöht, Erhalten der Temperatur im ersten Reaktionsraum durch weitere Zugabe von kälterem Substrat, wobei sich in einem zweiten Reaktionsraum, der oberhalb des ersten Reaktionsraums gelegen ist, durch die von den im Substrat enthaltenen methanogenen Bakterien produzierte Gärungswärme eine zweiten Temperatur im Bereich von 35 bis 40 °C einstellt und Rezirkulieren von Substrat aus dem zweiten Reaktionsraum in den ersten Reaktionsraum, wobei die Menge an zugegebenem Substrat und Rezirkulat so bemessen wird, dass die Temperaturen im ersten und zweiten Reaktionsraum konstant sind, d.h. eine gewünschte kleine konstante Temperaturdifferenz aufweisen.

Erfindungsgemäß wird das Substrat nur unvollständig, d.h. bewusst nicht auf das Niveau der eigentlichen Fermentationstemperatur, im ersten Reaktionsraum erwärmt. Vorzugsweise wird das Substrat auf eine Temperatur im Bereich von 0,1 bis 5 °C, besonders bevorzugt auf eine Temperatur im Bereich von 1 bis 3 °C unterhalb der Temperatur im ersten Reaktionsraum vorgewärmt.

Anschließend wird das vorgewärmte Substrat in den ersten Reaktionsraum eingebracht, wobei die Füllung des Reaktionsraumes vorzugsweise von unten nach oben erfolgt. Durch die im Substrat enthaltenen Bakterien wird während der Vergärung des organischen Materials die Temperatur im ersten Reaktionsraum auf den bevorzugten Bereich von 28 bis 33 °C, besonders bevorzugt auf den Bereich von 30 bis 33 °C angehoben. Dabei wird durch die kontinuierliche Zugabe von kälterem Substrat gewährleistet, dass die Temperatur im ersten Reaktionsraum nicht über den bevorzugten Temperaturbereich ansteigt. Dadurch werden insbesondere für die bereits im Substrat enthaltenen fermentativen und acetogenen Bakterien im ersten Reaktionsraum ideale Wachstumsbedingungen geschaffen.

Während der weiteren Zugabe von kälterem Substrat in den ersten Reaktionsraum bildet sich aufsteigend nach oben ein Temperaturgradient aus, wobei sich in dem zweiten Reaktionsraum eine zweite Temperatur im Bereich von 35 bis 40 °C, vorzugsweise im Bereich von 35 bis 38 °C einstellt. Mit zunehmender Wärme gewinnen die im Substrat enthaltenen methanogenen Bakterien die Oberhand im System und siedeln sich bevorzugt auf Aufwuchsträgern an. Durch die Wärmeproduktion der methanogenen Bakterien wird der Temperaturgradient im Bioreaktor aufrecht erhalten.

Ein gewisser Anteil an Substrat wird erfindungsgemäß aus dem zweiten Reaktionsraum in den ersten Reaktionsraum rezirkuliert, wobei die Menge und Temperatur an zugegebenem Substrat und Rezirkulat so bemessen wird, dass die Temperaturen im ersten und zweiten Reaktionsraum konstant bleiben. Dabei können Substrat und Rezirkulat gemeinsam zugeführt werden. Dabei wird die gemeinsam zurückgeführte Strecke hinsichtlich Verweilzeit und Temperaturführung so bemessen, dass speziell die im Zulauf enthaltenen gröberen Partikel nicht vollständig auf die Mischtemperatur gebracht werden können. Damit sind diese tendenziell kühler als die umgebende Flüssigkeit und werden somit bevorzugt in den unteren Bereich des ersten Reaktionsraumes transportiert. In dem dortigen Versäuerungsbereich werden die Partikel hydrolysiert und sind acetogenen Bakterien ausgesetzt. Als Folge des ablaufenden mikrobiellen Stoffumsatzes entstehen hierbei Säuren, wie Propionsäure, Valeriansäure, Essigsäure etc.. Der pH-Wert in dem Versäuerungsbereich liegt vorzugsweise im Bereich von 5,5 bis 6,5, besonders bevorzugt im Bereich von 5,8 bis 6,3. Bevorzugte Mikroorganismen für die Hydrolyse sind anaerobe oder fakultativ anaerobe fermentative Bakterien, wie beispielsweise *Escherichia coli.* Bei den acetogenen Bakterien handelt es sich vorzugsweise ebenfalls um fermentative Bakterien und acetogene Bakterien, wie Acetobacterien, insbesondere *A. woodii,* Bacteroide, Clostridien, wie *Clostridium aceticum* oder Syntrophobacter. Weitere geeignete Bakterien sind dem Fachmann bekannt. Besonders bevorzugt handelt es sich bei den Bakterien in dem Versäuerungsbereich um Mischpopulationen.

In dem Versäuerungsbereich wird das Substrat teilweise aufgeschlossen und gelangt nach einer Zeitverzögerung, die vor allem durch Anwärmen und Ausgleichen der Dichteunterschiede bedingt ist, in den oberen Methanisierungsbereich. Im Methanisierungsbereich herrscht neben der höheren Temperatur auch ein höherer pH-Wert. Vorzugsweise liegt der pH-Wert im Methanisierungsbereich im Bereich von 6,8 bis 8, besonders bevorzugt im Bereich von 7 bis 7,5. Bevorzugte Mikroorganismen für die Methanisierungsbereich sind vorzugsweise Bakterien der Klassen Methanobacteria, Methanococci, Methanosarcina und Methanomicrobia. Weitere geeignete Bakterien sind dem Fachmann bekannt. Besonders bevorzugt handelt es sich bei den Bakterien im Methanisierungsbereich um Mischpopulationen.

Erfindungsgemäß weisen Versäuerungsbereich und Methanisierungsbereich also unterschiedliche Temperaturen, unterschiedliche pH-Werte und unterschiedliche Mischpopulationen an Bakterien auf. Dabei müssen die Substrate im Versäuerungsbereich und im Methanisierungsbereich in der Regel nicht angeimpft werden, da in dem zugeführten Substrat ausreichend Biomasse vorhanden ist. Durch das erfindungsgemäße Verfahren wird dann das Wachstum von unterschiedlichen Bakteriengruppen gefördert. Um das Anfahren des Reaktors zu beschleunigen, kann allerdings mit Substrat eines bereits laufenden Reaktors oder Bewuchs von Aufwuchsträgern angeimpft werden.

In einer bevorzugten Ausgestaltung der Erfindung wird zum Vorwärmen des Substrats die Abwärme des Gärguts und/oder Gärrestes im Überlauf benutzt. Alternativ oder ergänzend kann auch die Abwärme des Rezirkulats verwendet werden.

In einer weiteren bevorzugten Ausgestaltung der Erfindung wird das Substrat im Verhältnis 1:1 bis 1:10, vorzugsweise im Verhältnis 1:5 bis 1:10 mit dem Rezirkulat vermischt. Dabei werden vorteilhafterweise Biomasse und noch nicht vergorenes Substrat zurückgeführt und die Temperatur des eingehenden Substrats geregelt. Zusätzlich kann über das Verhältnis der Reaktorprozess gesteuert werden, wobei auch auf Qualitäts- und Zusammensetzungsschwankungen des Substrats reagiert wird. Beispielsweise kann bei Rückgang der Gärleistung, z.B. aufgrund eingetragener Substratverunreinigungen wie Antibiotika, Desinfektionsmitteln oder anderen Hemmstoffen nur noch ein gewisser Teil des Frischsubstrates mit dem Rezirkulat vermischt werden. Der andere Teil wird direkt in den unteren konisch geformten Teil des Reaktors eingespeist. Auf diese Weise kommt es zu einer starken Abkühlung im konischen Teil des Reaktors mit der Folge einer noch stärker ausgeprägten Schichtung zwischen Versäuerungs- und Methanisierungsbereich. Die relative Verweilzeit des Substrates im Versäuerungsbereich steigt dadurch an mit dem Vorteil, dass in diesem Teil des Reaktors tendenziell einfach und schnell Hemmstoffe abgebaut und/oder neutralisiert werden können. Hierbei wird die Tatsache ausgenutzt, dass die säurebildenden Bakterien, die am Biogasprozess beteiligt sind, toleranter gegen verschiedene Störstoffe sind als die methanogenen Bakterien. Der Gärprozess erholt sich somit deutlich schneller nach einer Stoßbelastung, weil die langsam wachsenden methanogenen Bakterien nicht so stark von den Hemmstoffen beeinflusst werden, wie in klassischen einstufigen Reaktoren.

In einer weiteren bevorzugten Ausgestaltung der Erfindung umfasst das erfindungsgemäße Verfahren zusätzlich noch die Schritte Sedimentieren von aktiver Biomasse in aus dem Prozess entnommenen Gärresten und Rückführen der aktiven Biomasse in den ersten Reaktionsraum. Dadurch wird die einmal im Reaktor aufgebaute Biomasse kontinuierlich erhalten und vermehrt. Eine Verminderung der Biomasse ergibt sich lediglich durch absterbende Bakterien, die dann allerdings als Substrat mit vergoren werden.

Das erfindungsgemäße Verfahren eignet sich besonders gut für Substrate mit einem geringen Trockenmassen (TS-)Gehalt, wobei das Substrat vorzugsweise einen TS-Gehalt im Bereich von 1 bis 20%, besonders bevorzugt einen TS-Gehalt im Bereich von 1 bis 10%, weiter bevorzugt im Bereich von 2 bis 6% aufweist. Bevorzugte Substrate sind Gülle, insbesondere Schweine- oder Rindergülle, Melasse oder Vinasse, Glycerin, Stärke, Maisquellwasser oder Mischungen davon.

Das erfindungsgemäß Verfahren zeichnet sich vor allem durch extrem kurze Verweilzeiten bei gleichzeitig hohem Stoffumsatz aus, der auch mit kleinen Volumina erreicht wird.

Im Folgenden soll die Erfindung an Hand von Beispielen näher erläutert, aber keinesfalls auf diese beschränkt werden.

Es zeigen:
- Fig. 1: eine schematische Zeichnung der erfindungsgemäßen Vorrichtung mit einem Bioreaktor 11 und einem überstauten Feststoffabscheider 40
- Fig. 2: eine Vergrößerung des Bioreaktors 11.

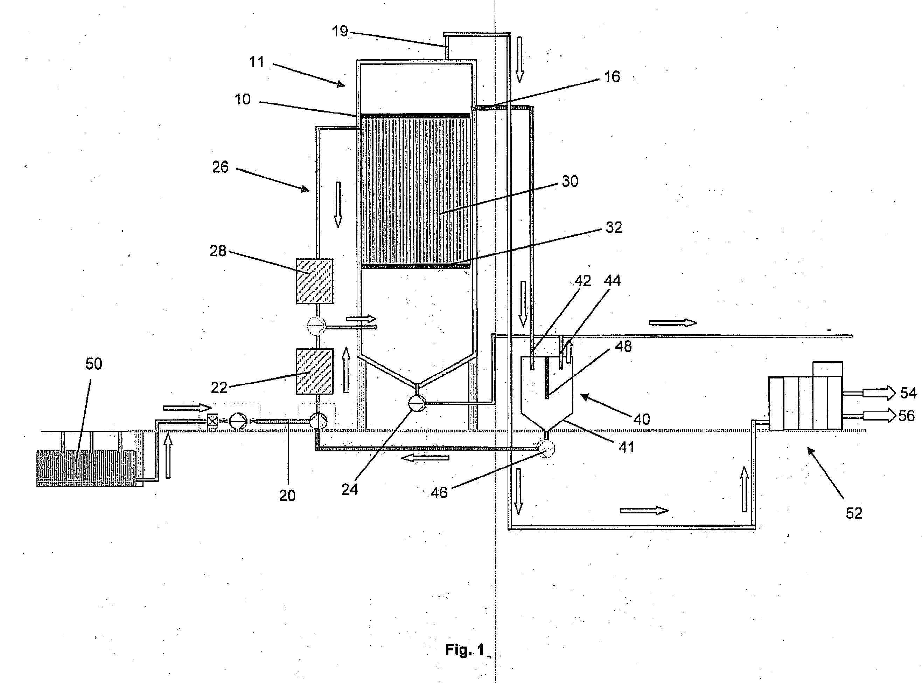

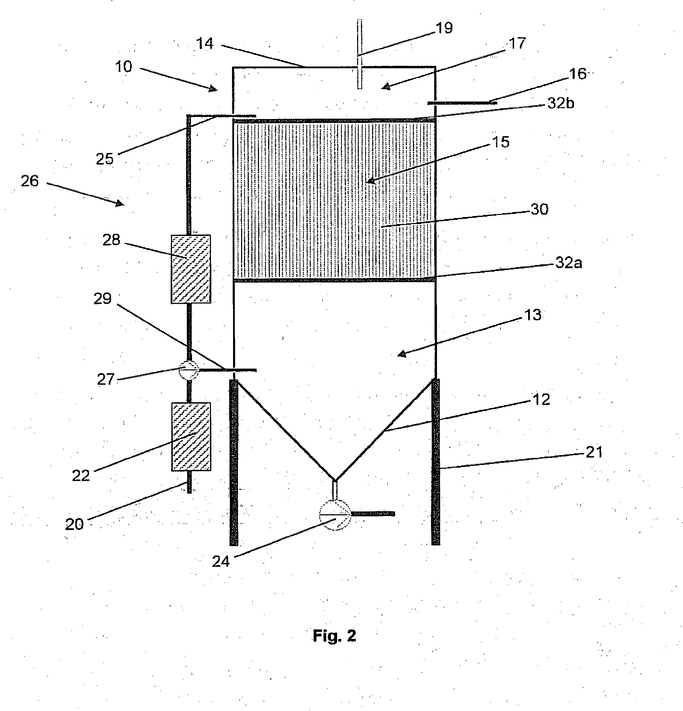

In Fig. 1 ist eine erfindungsgemäße Vorrichtung zur Vergärung von niederviskosen organischen Materialien gezeigt, wobei ein Bioreaktor 11 mit einem überstauten Feststoffabscheider 40 verbunden ist. Die Details des Bioreaktors 11 sind an Hand von Fig. 2 beschrieben. Der Bioreaktor 11 weist einen Reaktorbehälter 10 mit einem konusförmigen Reaktorboden 12 und einer Reaktordecke 14 und dazwischen liegenden Seitenwänden auf. Über Fußstützen 21 ist der Reaktorbehälter 10 auf einer Aufstellfläche aufgestellt, wobei die Längsachse des Behälters 10 senkrecht zur Aufstellfläche verläuft. Der Reaktorbehälter 10 hat eine kreisförmige Grundfläche mit einem Durchmesser von 8 m. Die Höhe der Seitenwände beträgt 19 m, so dass sich ein Volumen von 90 m³ ergibt. Auf einer Seitenwand unterhalb der Reaktordecke 14 ist ein Überlauf 16 angeordnet. Der Überlauf 16 ist über ein Ablaufrohr mit dem überstauten Feststoffabscheider 40 verbunden und dient der Entnahme von vergorenem Gärrest. Vorzugsweise ist der Überlauf 16 3 m unterhalb der Reaktordecke 14 angeordnet. Der Bereich vom Überlauf 16 bis zur Reaktordecke 14 stellt einen Gassammelraum 17 dar. In der Reaktordecke 14 ist ein Gasentnahmemittel 19 zur Abfuhr des produzierten Gases aus dem Gassammelraum 17 angeordnet. Mittels des Gasentnahmemittels 19 wird das im Bioreaktor 11 produzierte Gas in eine Blockheizkraftwerks (BHKW)-Einheit 52 überführt und dort zur Energiegewinnung von Strom 54 und Wärme 56 benutzt.

Im Inneren des Reaktorbehälters 10 sind zwei Aufnahmen 32 zur Befestigung einer Vielzahl von Aufwuchsträgern 30 angeordnet. Die Ausrichtung der Aufnahmen 32 ist parallel zur Reaktordecke 14. Die erste Aufnahme 32a befindet sich auf 35 % der Höhe der Seitenwände und die zweite Aufnahme 32b befindet sich auf 20 % der Höhe der Seitenwände jeweils bezogen auf den Abstand zur Reaktordecke 14. Der Bereich vom konusförmigen Boden 12 bis zur ersten Aufnahme 32a bildet einen ersten Reaktionsraum 13 und der Bereich von der ersten Aufnahme 32a bis zum Überlauf 16 bildet einen zweiten Reaktionsraum 15. Der konusförmige Reaktorboden 12 erstreckt sich über 10 % der Gesamthöhe des Reaktorbehälters 10. An der Spitze des Reaktorbodens 12 ist ein Feststoffentnahmemittel 24 angeordnet, durch das im Wesentlichen Inertmasse entnommen wird.

Zwischen den Aufnahmen 32 ist eine Vielzahl von Aufwuchsträgern 30 segeltuchförmig gespannt. Bei den Aufwuchsträgern 30 handelt es sich um Polypropylenfolien, mit einer veredelten Oberfläche. Zur Veredelung der Oberfläche der Polypropylenfolien wurden die Folien zuerst mikrostrukturiert und dann mit einer 10 %-igen Stärkelösung beschichtet. Die Mikrostrukturierung erfolgte mit einem 10 MeV Linearbeschleuniger bei 50 Hz, mit einer Pulsrate von 4 µs. Die eingestrahlte Gesamtdosis betrug 25 kGy. Der Abstand zwischen den einzelnen Folien beträgt 8 cm.

Im Bereich des ersten Reaktionsraumes 13 weist der Bioreaktor 11 eine Substratzufuhr 20 auf, die vorzugsweise im Bereich des konusförmigen Reaktorbodens 12 in den Reaktorbehälter 10 geführt wird. Die Substratzufuhr 20 weist weiterhin einen ersten Wärmetauscher 22 zur Temperierung des sich in der Substratzufuhr 20 befindenden Substrats auf. Das Substrat wird einem Güllelager 50 entnommen, durch den Wärmetauscher 22 und dann in den Reaktorbehälter 10 geführt.

Mittels eines Rezirkulationsmittels 26 wird Rezirkulat aus dem zweiten Reaktionsraum 15 in den ersten Reaktionsraum 13 überführt. Dafür weist das Rezirkulationsmittel 26 ein Aufnahmemittel 25 auf, durch das vergorenes Substrat aus dem zweiten Reaktionsraum 15 in das Rezirkulationsmittel 26 gelangt. Anschließend wird das Rezirkulat durch einen zweiten Wärmetauscher 28 zur Temperierung geführt. Nachdem das Rezirkulat temperiert ist, wird es über ein Abgabemittel 27 dem Reaktorbehälter 10 auf Höhe des ersten Reaktionsraums 13 wieder zugeführt. Substratzufuhr 20 und Rezirkulationsmittel 26 weisen optimalerweise eine gemeinsame Zuführung 29 auf, in der Substrat und Rezirkulat in beliebigem Verhältnis miteinander vermischt werden.

Das Aufnahmemittel 25 ist unterhalb des Überlaufs 16 auf den Seitenwänden des Reaktorsbehälters 10 angebracht, wobei Aufnahmemittel 25 und Überlauf 16 vorzugsweise auf derselben Seite wie die Substratzufuhr 20 angeordnet sind. Dadurch wird es möglich, den Überlauf 16 durch den Wärmetauscher 20 zu führen, um mittels der Abwärme des ablaufenden Gärrestes das Substrat vorzuwärmen.

Des Weiteren weist die erfindungsgemäße Vorrichtung einen überstauten Feststoffabscheider 40 auf. Bei dem überstauten Feststoffabscheider 40 handelt es sich ebenfalls um einen zylinderförmigen Behälter mit einem konusförmigen Boden 41. Das Volumen des Feststoffabscheiders 40 beträgt 2 m³. Der Feststoffabscheider 40 ist am tiefsten Punkt des Reaktorbehälters 10 neben diesem angeordnet. Vom Überlauf 16 des Reaktorbehälters 10 führt eine Verbindung direkt in einen Zulauf 42 des Feststoffabscheiders 40. Im Inneren des Feststoffabscheiders 40 ist eine Trennwand 48 angeordnet, die den Zulauf 42 von einem Ablauf 44 trennt. Dadurch wird ein Kurzschluss zischen den ein- und abgehenden Stoffströmen vermieden. Die Trennwand 48 reicht bis zu ²/₃in den Behälter des überstauten Feststoffabscheiders 40 hinein. In dem Feststoffabscheider 40 sinkt aktive Biomasse auf den Boden und kann durch ein Feststoffentnahmemittel 46 entnommen werden und über die Substratzufuhr 20 wieder dem ersten Reaktionsraum 13 zugeführt werden. Tote Biomasse und Inertmasse stellen den Gärrest dar und werden durch den Ablauf 44 der Endlagerung zugeführt.

### Beispiel 1

Eine erfindungsgemäße Vorrichtung wurde zur Vergärung von niederviskosem organischem Material eingesetzt. Schweinegülle mit einem TS-Gehalt von 4,5 % wurde als Substrat bei 15 °C in einem Güllelager 50 gelagert. Mit einer Pumpgeschwindigkeit von 20 I/min wurde die Schweinegülle als Substrat in den Bioreaktor 11 gepumpt. In dem ersten Wärmetauscher 22 erfolgte eine Erwärmung der Gülle bis auf 25 °C. In der Auffüllphase des Reaktors musste extern zugeheizt werden; unter laufendem Betrieb war die Abwärme des Gärrestes im Überlauf 16 ausreichend. Im ersten Reaktionsraum 13 bildet sich der Versäuerungsbereich mit einer Temperatur T₁ = 32 °C und einem pH-Wert von 5,8-6,3. In diesem Bereich herrschen Hydrolyse und Acetogenese vor. Im zweiten Reaktionsraum 15 bildet sich der Methanisierungsbereich mit einer Temperatur T₂ = 36 °C und einem pH-Wert von 7,0 bis 7,5 aus. In diesem Bereich ist die Methanbildung der vorherrschende Stoffwechselvorgang. Nach Füllung des Reaktorbehälters 10 wurde die Pumpgeschwindigkeit für das Substrat auf 10 I/min reduziert. Gleichzeitig wurde über das Rezirkulationsmittel Rezirkulat mit einer Geschwindigkeit von 70 I/min aus dem zweiten Reaktionsraum 15 in den ersten Reaktionsraum 13 überführt. Dabei wurden Substrat und Rezirkulat in einem Verhältnis von 1:7 in der gemeinsamen Zufuhr 29 miteinander vermischt. Nach Ausbildung der Kompartimentierung konnte der Reaktor über mehrere Tage stabil betrieben werden. Innerhalb von 3 Tagen konnte bezogen auf die Trockenmasse der Schweinegülle eine Abbaurate von etwa 80 % erreicht werden.

### Bezugszeichenliste

- 10: Reaktorbehälter
- 11: Bioreaktor
- 12: Reaktorboden
- 13: erster Reaktionsraum
- 14: Reaktordecke
- 15: zweiter Reaktionsraum
- 16: Überlauf
- 17: Gassammelraum
- 24,46: Feststoffentnahmemittel
- 19: Gasentnahmemittel
- 20: Substratzufuhr
- 21: Fußstützen
- 22, 28: Wärmetauscher
- 25: Aufnahmemittel
- 26: Rezirkulationsmittel
- 27: Abgabemittel
- 29: Zuführung
- 30: Aufwuchsträger
- 32, 32a, 32b: Aufnahme
- 40: überstauter Feststoffabscheider
- 41: konusförmiger Boden
- 42: Zulauf
- 44: Ablauf
- 48: Trennwand
- 50: Güllelager
- 52: BHKW-Einheit
- 54: Strom
- 56: Wärme

## Patentansprüche

1. Vorrichtung zur Vergärung von niederviskosen organischen Materialien aufweisend einen Bioreaktor (11) und mindestens einen überstauten Feststoffabscheider (40),
wobei der Bioreaktor (11) besteht aus:
- einem Reaktorbehälter (10) mit einem konusförmigen Reaktorboden (12) mit Feststoffentnahmemittel (24) zur Entnahme des Gärrestes, Seitenwänden und einer Reaktordecke (14), wobei im Inneren des Reaktorbehälters (10) mindestens eine Aufnahme (32) zur Befestigung einer Vielzahl von Aufwuchsträgern (30) im Wesentlichen parallel zur Reaktordecke (14) und im mittleren Drittel des Reaktorbehälters (10) angeordnet ist, und wobei der Bereich vom konusförmigen Boden (12) bis zur mindestens einen Aufnahme (32) einen ersten Reaktionsraum (13) und der Bereich von der mindestens einen Aufnahme (32) bis zur Reaktordecke (14) einen zweiten Reaktionsraum (15) bildet,
- einer Vielzahl von Aufwuchsträgern (30), die von der Reaktordecke (14) bis zur mindestens einen Aufnahme (32) gespannt, sind,
- mindestens einer Substratzufuhr (20) im Bereich des ersten Reaktionsraumes (13), wobei die Substratzufuhr (20) außerhalb des Reaktorbehälters (10) einen ersten Wärmetauscher (22) zur Temperierung des sich in der Substratzufuhr (20) befindenden Substrats aufweist,
- mindestens einem Rezirkulationsmittel (26), das geeignet ist, Rezirkulat aus dem zweiten Reaktionsraum (15) in den ersten Reaktionsraum (13) zu überführen, wobei das Rezirkulationsmittel (26) ein Mittel (25) zur Aufnahme des Rezirkulats und ein Mittel (27) zur Abgabe des Rezirkulats aufweist sowie einen zweiten Wärmetauscher (28) zur Temperierung des sich in dem Rezirkulationsmittel (26) befindenden Rezirkulats, wobei Rezirkulationsmittel (26) und Wärmetauscher (28) außerhalb des Reaktorbehälters (10) angeordnet sind,
- mindestens einem Überlauf (16), der im Bereich des zweiten Reaktionsraums (15) zwischen der Reaktordecke (14) und dem Rezirkulationsmittel (26) angeordnet ist, wobei der zwischen Reaktordecke (14) und Überlauf (16) befindliche Teil des zweiten Reaktionsraumes (15) ein Gassammelraum (17) ist, in dessen Bereich ein Gasentnahmemittel (19) angeordnet ist, und
wobei der mindestens eine überstaute Feststoffabscheider (40)
- einen konusförmigen Boden (41) aufweist, wobei an der Spitze des Konus (41) ein Feststoffentnahmemittel (46) angeordnet ist, das mit der Substratzufuhr (20) des Bioreaktors (11) verbunden ist, und
- mindestens einen Zulauf (42) aufweist, der mit dem Überlauf (16) des Bioreaktors (11) verbunden ist,
und wobei der überstaute Feststoffabscheider (40) auf Höhe oder unterhalb des Reaktorbodens (12) neben dem Bioreaktor (11) angeordnet ist.

2. Vorrichtung nach Anspruch 1 , **dadurch gekennzeichnet, dass** das Mittel (25) zur Aufnahme des Rezirkulats im Bereich von 0 bis 20% der Höhe des Reaktorbehälters (10) und das Mittel (27) zur Abgabe des Rezirkulats im Bereich von 60 bis 80%, vorzugsweise im Bereich von 70 bis 80% der Höhe des Reaktorbehälters (10), jeweils bezogen auf die Reaktordecke (14), angeordnet ist.

3. Vorrichtung nach Anspruch 1 oder 2, **dadurch gekennzeichnet, dass** die Substratzufuhr (20) und das Mittel zur Abgabe des Rezirkulats (27) eine gemeinsame Zuführung (29) in den Reaktorbehälter (10) aufweisen.

4. Vorrichtung nach einem der Ansprüche 1 bis 3, **dadurch gekennzeichnet, dass** die Aufwuchsträger (30) aus Kunststofffolien, vorzugsweise aus Polyethylen, Polypropylen, Polystyrol, Polyvinylchlorid, Polyamin, Polyimid oder Mischungen daraus, besonders bevorzugt aus Polyethylen oder Polypropylen bestehen.

5. Vorrichtung nach Anspruch 4, **dadurch gekennzeichnet, dass** die Aufwuchsträger (30) mikrostrukturiert und/oder mit organischen Substraten, vorzugsweise mit Stärke und/oder Stärkederivaten, beschichtet sind.

6. Vorrichtung nach einem der Ansprüche 1 bis 5, **dadurch gekennzeichnet, dass** die Substratzufuhr (20) im Bereich von 15 bis 40%, vorzugsweise im Bereich von 15 bis 30% der Höhe des Reaktorbehälters (10) bezogen auf den Reaktorboden (12) angeordnet ist.

7. Verfahren zur Vergärung von niederviskosen organischen Materialien unter Verwendung einer Vorrichtung nach einem der Ansprüche 1 bis 6, umfassend die Schritte:
- Vorwärmen der niederviskosen organischen Materialien auf eine Temperatur unterhalb einer Temperatur (T₁) im Bereich von 28 bis 33 °C,
- Einbringen der vorgewärmten niederviskosen organischen Materialien als Substrat in den ersten Reaktionsraum (13) des Bioreaktors (11), wobei sich durch die von den im Substrat enthaltenen acetogenen Bakterien produzierte Gärungswärme im ersten Reaktionsraum (13) die Temperatur auf die Temperatur (T₁) erhöht,
- Erhalten der Temperatur (T₁) im ersten Reaktionsraum (13) durch weitere Zugabe von kälterem Substrat, wobei sich im zweiten Reaktionsraum (15), der oberhalb des ersten Reaktionsraums (13) gelegen ist, durch die von den im Substrat enthaltenen methanogenen Bakterien produzierte Gärungswärme eine zweiten Temperatur (T₂) im Bereich von 35 bis 40 °C einstellt, und
- Rezirkulieren von Substrat aus dem zweiten Reaktionsraum (15) in den ersten Reaktionsraum (13), wobei die Menge an zugegebenem Substrat und Rezirkulat so bemessen wird, dass die Temperaturen (T₁) und (T₂) im ersten und zweiten Reaktionsraum (13, 15) konstant sind.

8. Verfahren nach Anspruch 7, **dadurch gekennzeichnet, dass** im ersten Reaktionsraum (13) eine Temperatur (T₁) im Bereich von 30 bis 33 °C und/oder im zweiten Reaktionsraum (15) eine Temperatur (T₂) im Bereich von 35 bis 38 °C eingestellt wird.

9. Verfahren nach Anspruch 7 oder 8, **dadurch gekennzeichnet, dass** das Substrat auf eine Temperatur im Bereich von 0,1 bis 5 °C, vorzugsweise auf eine Temperatur im Bereich von 1 bis 3 °C unterhalb der ersten Temperatur (T₁) vorgewärmt wird.

10. Verfahren nach einem der Ansprüche 7 bis 9, **dadurch gekennzeichnet, dass** zum Vorwärmen des Substrats die Abwärme des im Überlauf abgeführten Gärrestes verwendet wird.

11. Verfahren nach einem der Ansprüche 7 bis 10, **dadurch gekennzeichnet, dass** das Substrat im Verhältnis 1:1 bis 1:10, vorzugsweise im Verhältnis 1:5 bis 1:10 mit dem Rezirkulat vermischt wird.

12. Verfahren nach einem der Ansprüche 7 bis 11, **dadurch gekennzeichnet, dass** das Substrat einen Trockenmassen- (TS)-gehalt im Bereich von 1 bis 20%, vorzugsweise im Bereich von 1 bis 10%, weiter bevorzugt im Bereich von 2 bis 6% aufweist.

## Claims

1. A device for fermenting low-viscosity organic materials, having a bioreactor (11) and at least one overstowed solid separator (40),
wherein the bioreactor (11) consists of:
- a reactor container (10) with a conical reactor bottom (12) with solid removal means (24) for removing the fermentation residue, side walls and a reactor cover (14), wherein at least one receptacle (32) for fastening a multiplicity of growth supports (30) is arranged in the interior of the reactor container (10) essentially parallel to the reactor cover (14) and in the central third of the reactor container (10), and wherein the region from the conical bottom (12) to the at least one receptacle (32) forms a first reaction space (13) and the region from the at least one receptacle (32) to the reactor cover (14) forms a second reaction space (15),
- a multiplicity of growth supports (30), which are stretched from the reactor cover (14) to at least one receptacle (32),
- at least one substrate supply (20) in the region of the first reaction space (13), wherein the substrate supply (20) has a first heat exchanger (22) outside of the reactor container (10) for temperature control of the substrate located in the substrate supply (20),
- at least one recirculation means (26), which is suitable for transferring recirculated material from the second reaction space (15) to the first reaction space (13), wherein the recirculation means (26) has a means (25) for accommodating the recirculated material and a means (27) for outputting the recirculated material and also a second heat exchanger (28) for the temperature control of the recirculated material located in the recirculation means (26), wherein recirculation means (26) and heat exchanger (28) are arranged outside of the reactor container (10),
- at least one overflow (16), which is arranged in the region of the second reaction space (15) between the reactor cover (14) and the recirculation means (26), wherein the part of the second reaction space (15) located between reactor cover (14) and overflow (16) is a gas collection space (17), in the region of which, a gas removal means (19) is provided, and
wherein the at least one overstowed solid separator (40)
- has a conical bottom (41), wherein a solid removal means (46) is arranged at the tip of the cone (41), which is connected to the substrate supply (20) of the bioreactor (11), and
- has at least one feed (42), which is connected to the overflow (16) of the bioreactor (11),
and wherein the overstowed solid separator (40) is arranged at the height of or below the reactor bottom (12) next to the bioreactor (11).

2. The device according to Claim 1, **characterised in that** the means (25) for accommodating the recirculated material is arranged in the region of 0 to 20% of the height of the reactor container (10) and the means (27) for outputting the recirculated material is arranged in the region of 60 to 80%, preferably in the region of 70 to 80% of the height of the reactor container (10), in each case with respect to the reactor cover (14).

3. The device according to Claim 1 or 2, **characterised in that** the substrate supply (20) and the means for outputting the recirculated material (27) have a common supply (29) into the reactor container (10).

4. The device according to one of Claims 1 to 3, **characterised in that** the growth supports (30) consist of plastic films, preferably of polyethylene, polypropylene, polystyrene, polyvinyl chloride, polyamide, polyimide or mixtures thereof, particularly preferably of polyethylene or polypropylene.

5. The device according to Claim 4, **characterised in that** the growth supports (30) are microstructured and/or coated with organic substrates, preferably with starch and/or starch derivatives.

6. The device according to one of Claims 1 to 5, **characterised in that** the substrate supply (20) is arranged in the region of 15 to 40%, preferably in the region of 15 to 30% of the height of the reactor container (10) with respect to the reactor bottom (12).

7. A method for fermenting low-viscosity organic materials using a device according to one of Claims 1 to 6, comprising the steps:
- preheating the low-viscosity organic materials to a temperature below a temperature (T₁) in the range from 28 to 33°C,
- introducing the preheated low-viscosity organic materials as substrate into the first reaction space (13) of the bioreactor (11), wherein the temperature increases to the temperature (T₁) due to the fermentation heat produced in the first reaction space (13) by the acetogenic bacteria contained in the substrate,
- obtaining the temperature (T₁) in the first reaction space (13) by further addition of colder substrate, wherein a second temperature (T₂) in the range from 35 to 40°C is set in the second reaction space (15), which is placed above the first reaction space (13), due to the fermentation heat produced by the methanogenic bacteria contained in the substrate, and
- recirculating substrate from the second reaction space (15) into the first reaction space (13), wherein the amount of added substrate and recirculated material is measured such that the temperatures (T₁) and (T₂) in the first and second reaction spaces (13, 15) are constant.

8. The method according to Claim 7, **characterised in that** a temperature (T₁) in the first reaction space (13) in the range from 30 to 33°C and/or a temperature (T₂) in the second reaction space (15) in the range from 35 to 38°C is set.

9. The method according to Claim 7 or 8, **characterised in that** the substrate is preheated to a temperature in the range from 0.1 to 5°C, preferably in the range from 1 to 3°C below the first temperature (T₁).

10. The method according to one of Claims 7 to 9, **characterised in that** the waste heat of the fermentation residue dissipated in the overflow is used for preheating the substrate.

11. The method according to one of Claims 7 to 10, **characterised in that** the substrate is mixed with the recirculated material in a ratio 1:1 to 1:10, preferably in the ratio 1:5 to 1:10.

12. The method according to one of Claims 7 to 11, **characterised in that** the substrate has a dry mass (TS) content in the range from 1 to 20%, preferably in the range from 1 to 10%, further preferably in the range from 2% to 6%.

## Revendications

1. Procédé de fermentation de matières organiques de faible viscosité, comportant un bioréacteur (11) et au moins un séparateur de graisses (40) submergé,
le bioréacteur (11) étant constitué :
- d'une cuve (10) de réacteur avec un fond inférieur de réacteur (12) de forme conique, avec des moyens de prélèvement (24) de solides pour prélever les restes de fermentation, des parois latérales et un fond supérieur (14) de réacteur, à l'intérieur de la cuve (10) du réacteur étant placé au moins un logement (32) pour la fixation d'une pluralité de supports d'accroissance (30), sensiblement à la parallèle du fond supérieur (14) de réacteur et dans le tiers central de la cuve (10) du réacteur et la région du fond inférieur (12) conique jusqu'à l'au moins un logement (32), formant un premier espace de réaction (13) et la région de l'au moins un logement (32) jusqu'au fond supérieur (14) du réacteur formant un second logement de réaction (15).
- d'une pluralité de supports d'accroissement (30), qui sont tendus du fond supérieur (14) du réacteur jusqu'à l'au moins un logement (32),
- d'au moins un amenage de substrat (20), dans la région du premier espace de réaction (13), l'amenage de substrat (20) comportant à l'extérieur de la cuve (10) de réacteur un premier échangeur thermique (22), destiné à tempérer le substrat qui se trouve dans l'amenage de substrat (20),
- d'au moins un moyen de recirculation (26), qui est capable de transférer du recirculé du second espace de réaction (15) dans le premier espace de réaction (13), le moyen de recirculation (26) comportant un moyen (25) pour recevoir le recirculé et un moyen (27) pour restituer le recirculé, ainsi qu'un second échangeur thermique (28), destiné à tempérer le recirculé qui se trouve dans le moyen de recirculation (26), les moyens de recirculation (26) et l'échangeur thermique (28) étant placés à l'extérieur de la cuve (10) du réacteur,
- d'au moins un trop-plein (16) qui est placé dans la région du second espace de réaction (15), entre le fond supérieur (14) du réacteur et le moyen de recirculation (26), la partie du second espace de réaction (15) qui se trouve entre le fond supérieur (14) du réacteur et le trop-plein (16) étant un espace collecteur de gaz (17), dans la région duquel est placé un moyen de prélèvement (19) de gaz,
l'au moins un séparateur de graisse (40) submergé
- comportant un fond inférieur (41) conique, à la pointe du cône (41) étant placé un moyen de prélèvement (46) de graisse qui est relié avec l' amenage de substrat (20) du bioréacteur (11) et
- comportant au moins une arrivée (42) qui est reliée avec le trop-plein (16) du bioréacteur (11),
et le séparateur de graisse (40) submergé étant placé au niveau ou sous le fond inférieur (12) du réacteur, à côté du bioréacteur (11).

2. Dispositif selon la revendication 1, **caractérisé en ce que** le moyen (25) destiné à recevoir le recirculé est placé dans la plage de 0 à 20 % de la hauteur de la cuve (10) de réacteur et le moyen (27) destiné à restituer le recirculé est placé dans la plage de 60 à 80 %, de préférence dans la plage de 70 à 80 % de la hauteur de la cuve (10) de réacteur, chaque fois en rapport au fond supérieur (14) du réacteur.

3. Dispositif selon la revendication 1 ou la revendication 2, **caractérisé en ce que** l'amenage de substrat (20) et le moyen destiné à restituer le recirculé (27) comportent une alimentation (29) commune dans la cuve (10) du réacteur.

4. Dispositif selon l'une quelconque des revendications 1 à 3, **caractérisé en ce que** les supports d'accroissance (30) sont constitués de films de matière plastique, de préférence de polyéthylène, de polypropylène, de polystyrène, de chlorure de polyvinyle, de polyamide, de polyimide ou de mélanges de ces derniers, de manière particulièrement préférée de polyéthylène ou de polypropylène.

5. Dispositif selon la revendication 4, **caractérisé en ce que** les supports d'accroissance (30) sont microstructurés et/ou revêtus de substrats organiques, de préférence d'amidon et/ou de dérivés d'amidon.

6. Dispositif selon l'une quelconque des revendications 1 à 5, **caractérisé en ce que** l'amenage de substrat (20) se trouve dans la plage de 15 à 40 %, de préférence dans la plage de 15 à 30 % de la hauteur de la cuve (10) de réacteur, en rapport au fond inférieur (12) du réacteur.

7. Procédé de fermentation de matières organiques de faible viscosité en utilisant un dispositif selon l'une quelconque des revendications 1 à 6, comprenant les étapes :
- préchauffage des matières organiques de faible viscosité à une température inférieure à une température (T₁), dans l'ordre de 28 à 33 °C,
- introduction des matières organiques de faible viscosité préchauffées en tant que substrat dans le premier espace de réaction (13) du bioréacteur (11), du fait de la chaleur de fermentation produite par les bactéries acétogènes contenues dans le substrat, la température s'élevant à la température (T₁) dans le premier espace de réaction (13),
- maintien de la température (T1) dans le premier espace de réaction (13), par ajout supplémentaire de substrat plus froid, dans le second espace de réaction (15) qui est situé au-dessus du premier espace de réaction (13), du fait de la chaleur de fermentation produite par les bactéries acétogènes contenues dans le substrat, s'établissant une deuxième température (T₂), dans l'ordre de 35 à 40 °C, et
- recirculation de substrat du second espace de réaction (15) dans le premier espace de réaction (13), la quantité de substrat ajouté et de recirculé étant dimensionnée de sorte que les températures (T₁) et (T₂) dans le premier et dans le second espaces de réaction (13, 15) soient constantes.

8. Procédé selon la revendication 7, **caractérisé en ce que** dans le premier espace de réaction (13), on règle une température (T₁) dans l'ordre de 30 à 33 °C et/ou dans le second espace de réaction (15), on règle une température (T₂) dans l'ordre de 35 à 38 °C.

9. Procédé selon la revendication 7 ou la revendication 8, **caractérisé en ce qu'**on préchauffe le substrat à une température inférieure dans l'ordre de 0,1 à 5 °C, de préférence à une température inférieure dans l'ordre de 1 à 3 °C à la première température (T₁).

10. Procédé selon l'une quelconque des revendications 7 à 9, **caractérisé en ce que** pour le préchauffage du substrat, on utilise la chaleur perdue du reste de fermentation évacué dans le trop-plein.

11. Procédé selon l'une quelconque des revendications 7 à 10, **caractérisé en ce qu'**on mélange le substrat dans la proportion de 1 : 1 à 1 : 10, de préférence dans la proportion de 1 : 5 à 1 : 10 avec le recirculé.

12. Procédé selon l'une quelconque des revendications 7 à 11, **caractérisé en ce que** le substrat présente une teneur en masse sèche (TS) dans l'ordre de 1 à 20%, de préférence dans l'ordre de 1 à 10 %, de manière plus préférée, dans l'ordre de 2 à 6 %.
